Europäisches Patentamt

European Patent Office

Office européen des brevets

(11) Publication number: **0 687 685 A1**

## EUROPEAN PATENT APPLICATION
## published in accordance with Art. 158(3) EPC

(21) Application number: **94908491.7**

(22) Date of filing: **03.03.94**

(86) International application number:
**PCT/JP94/00345**

(87) International publication number:
**WO 94/20531 (15.09.94 94/21)**

(51) Int. Cl.⁶: **C07K 7/06**, C07K 7/08,
A61K 37/64

(30) Priority: **03.03.93 JP 42608/93**
**27.01.94 JP 7662/94**

(43) Date of publication of application:
**20.12.95 Bulletin 95/51**

(84) Designated Contracting States:
**AT BE CH DE ES FR GB IT LI NL SE**

(71) Applicant: **TEIJIN LIMITED**
**6-7, Minamihommachi 1-chome**
**Chuo-ku**
**Osaka-shi,**
**Osaka 541 (JP)**

(72) Inventor: **KAWAMURA, Takashi, Tokyo**
**Research Center**
**Teijin Limited,**
**3-2, Asahigaoka 4-chome**
**Hino-shi,**
**Tokyo 191 (JP)**
Inventor: **NAKADA, Tomohisa, Tokyo**
**Research Center**
**Teijin Limited,**
**3-2, Asahigaoka 4-chome**
**Hino-shi,**
**Tokyo 191 (JP)**
Inventor: **SATO, Hideyuki, Tokyo Research**
**Center**
**Teijin Limited,**
**3-2, Asahigaoka 4-chome**
**Hino-shi,**
**Tokyo 191 (JP)**
Inventor: **KAWABATA, Hiroshi, Tokyo**
**Research Center**
**Teijin Limited,**
**3-2, Asahigaoka 4-chome**

**Hino-shi,**

**Tokyo 191 (JP)**

Inventor: **KAMIMURA, Takashi, Tokyo**

**Research Center**

**Teijin Limited,**

**3-2, Asahigaoka 4-chome**

**Hino-shi,**

**Tokyo 191 (JP)**

Inventor: **KUNORI, Yuichi, Tokyo Research**

**Center**

**Teijin Limited,**

**3-2, Asahigaoka 4-chome**

**Hino-shi,**

**Tokyo 191 (JP)**

Inventor: **KANNO, Hideo, Parknova Yokohama**

**Yoshinocho 204**

**1-2, Takasago-cho,**

**Minami-ku**

**Yokohama-shi,**

**Kanagawa 232 (JP)**

(74) Representative: **Votier, Sidney David**

**CARPMAELS & RANSFORD**

**43, Bloomsbury Square**

**London WC1A 2RA (GB)**

(54) **PHYSIOLOGICALLY ACTIVE PEPTIDE**

(57) A physiologically active peptide having the amino acid sequence represented by the following general formula (I): $A-W^1-X-V^1-Z-W^2-Y^1-V^2-Y^2-B$, wherein A and/or B contains a Cys residue that is modified and/or forms a disulfide linkage. A represents a peptide composed by one to six amino acid residues; B represents a peptide composed of one to three amino acid residues; $W^1$ and $W^2$ represent each independently Ser or Thr; X represents Lys or Arg; $V^1$ and $V^2$ represent each independently Ser, Arg, Thr, Tyr, Phe or Lys; $Y^1$ and $Y^2$ represent each independently Phe, Tyr or Trp; and Z represents Leu, Val, Ile, Pro, Phe or Ala.

## Fig.9

FIELD OF THE ART

The present invention relates to physiologically active peptides. More particularly, the present invention relates to peptides having an activity to inhibit binding of phospholipase $A_2$ and heparin, and an activity to inhibit phospholipase $A_2$.

BACKGROUND ART

Phospholipase $A_2$ ($PLA_2$) are enzymes which hydrolyze an ester bond at the 2-position of a phospholipid in the cell membrane, and include two members, i.e., pancreatic $PLA_2$ and non-pancreatic $PLA_2$. The pancreatic $PLA_2$ (type II $PLA_2$) liberates arachidonic acid (AA) from the phospholipid. The AA is converted to prostagrandins, tromboxanes and leucotrienes, which are physiologically active substances causing various inflammatory diseases and allergic diseases.

On the other hand, $PLA_2$ degrades phospholipids so as to destroy the cell membrane resulting in formation of lysolecithin having strong cytotoxicity. Recently, the pancreatitis caused by cell membrane injury action and its progress to higher severity and multi-organ injuries has attracted attention. In addition, reportedly the non-pancreatic $PLA_2$ is involved in these diseases. Accordingly, it is considered that since the liberation of AA, which is a precursor for various physiologically active substances, can be blocked by inhibitors of $PLA_2$, the inhibition of $PLA_2$ is useful for prophylactic and/or therapeutic treatments of various inflammatory diseases and allergic diseases. In addition, it is considered that since the cell membrane injury action can be inhibited, it is useful for prophylactic and/or therapeutic treatment of the pancratitis and its progress to higher severity as well as milti-organ injuries.

Further, biosynthesis of both prostograndins and leucotrienes can be suppressed by inhibiting $PLA_2$ activity, and therefore strong antiinflammatory activity and antiallergic activity with side-effects lower than those of present steroid-type drugs and non-steroid-type drugs are expected.

Heparin is a sulfated polysaccharide comprising D-N-acetylglucosamin and D-glucuronic acid or L-iduronic acid wherein N-acetyl groups in a substantial part of the glycoside chains are replaced with N-sulfate groups, and the hydroxyl groups in a substantial part of the glycoside chains are replaced with sulfate groups. Heparin and heparane sulfate having a structure similar to that of heparin have various physiological properties such as an inhibitory activity for blood clotting. According to recent research, since heparin binds to a series of cell growth factors called fibroblast growth factor (FGF) and is involved in transmission of FGF to tissues or cells, it was clarified that heparin sulfate is involved in the control of cell growth (M. Roghhani and D. Moscatelli, J. Biol. Chem., 267, 22156 to 22162 (1992)). Recently, type II phospholipase $A_2$ was purified from inflammatory sites in humans having inflammatory disease and in inflammatory model animals, and the properties thereof were clarified (Ichiro Kudo, Keizo Inoue, Seikagaku, 64, p. 1330 - 1334 (1992)). This enzyme is considered to have an action to promote an inflammatory response and therefore a substance inhibiting this enzyme is expected to exhibit an antiinflammatory action. In fact, type II phospholipase $A_2$ has a strong affinity to heparin, and is extra-cellularly secreted when an inflammation occurs. In addition, it is known that for the physiological activities of the type II phospholipase $A_2$ to be effective, it must be incorporated into tissue or cells, and for the incorporation heparin and heparan sulfate play an important role (Keizo Inoue et al., Jikken Igaku, 11, p. 1460 (1993)).

Accordingly, a substance which inhibits binding of type II phospholipase $A_2$ and heparin etc. is expected to exhibit an anti-inflammatory action.

WO93/01215 describes phospholipase $A_2$ inhibitors comprising 5 amino acids in the amino acid sequence of phospholipase $A_2$. However, it does not describe modification of Cys residue in claimed polypeptide to enhance physiological activities of the polypeptide.

DISCLOSURE OF THE INVENTION

The present inventors determined a region in type II phospholipase $A_2$, especially human type II phospholipase $A_2$, which has heparin-binding activity, and found that this peptide having heparin-binding ability inhibits the binding of human type II phospholipase $A_2$ and heparin, and that this peptide inhibits an enzymatic activity of human type II phospholipase $A_2$ in the presence of heparin, as well as that said activity is dramatically enhanced by a certain modification at the Cys residue of the peptide. On the basis of these findings, as a result of diligent research, the present inventors completed the present invention.

Accordingly the present invention provides a physiologically active peptide represented by the following formula (I):

A-W$^1$-X-V$^1$-Z-W$^2$-Y$^1$-V$^2$-Y$^2$-B    (I)

wherein A represents a peptide consisting of 1 to 6 amino acid residues; B represents a peptide consisting of 1 to 3 amino acid residues; W$^1$ and W$^2$ represent, independently, Ser or Thr; X represents Lys or Arg; V$^1$ and V$^2$ represent, independently, Ser, Arg, Thr, Tyr, Phe or Lys; Y$^1$ and Y$^2$ represent, independently, Phe, Tyr or Trp; and Z represents Leu, Val, Ile, Pro, Phe or Ala; at least one of A and B contains at least one Cys residue; and wherein

(1) in a peptide containing at least one Cys residue in A and/or B, said at least one Cys residue is modified at its -SH group;

(2) in a peptide containing at least one Cys residue in both of A and B, a ring structure has been formed by a disulfide bond between said two Cys resides;

(3) in more than one peptide each containing at least one Cys residue in at least one of A and/or B, a linear polymer has been formed by one or more disulfide bond formed between a Cys residue contained in A or B of one peptide and a Cys residue contained in A or B of another peptide;

(4) in more than one peptide each containing at least one Cys residue in both of A and B, a polymeric ring structure has been formed by more than one disulfide bond formed between a Cys residue contained in A or B of a peptide and Cys residue contained in A or B of another peptide; or

(5) in the structures (1) to (4), at least one Cys residue which does not form a disulfide bond has been modified at its -SH group.

BRIEF EXPLANATION OF DRAWINGS

Figure 1 shows a result of a reversed phase HPLC for peptide (2) in Example 1.2.

Fig. 2 shows a result of a reversed phase HPLC for peptide (11) in Example 3.2.

Fig. 3 shows a result of a reversed phase HPLC for peptide (16) in Example 3.3.

Fig. 4 shows a result of a reversed phase HPLC for peptide (13) in Example 4.2.

Fig. 5 shows a result of a reversed phase HPLC for peptide (21) in Example 5.

Fig. 6 shows a result of a reversed phase HPLC for peptide (22) in Example 8.

Fig. 7 shows a result of a reversed phase HPLC for peptide (17) in Example 6.

Fig. 8 shows a result of a reversed phase HPLC for peptides (16) and (17) in Example 6.

Fig. 9 shows a result of an assay for inhibition of binding of heparin and human type II PLA$_2$. In the figure, the circles show a result for peptide (20) of the present invention, and the quadrilaterals show a result for a control (Gly-Ala-Gly-Ala).

SPECIFIC EMBODIMENT FOR CARRYING OUT THE INVENTION

In the formula (I), at least one of A and B contains at least one Cys residue.

In the formula (I), A represents a peptide consisting of 1 to 6 amino acid residues. Amino acids forming A are, as a rule, L-amino acids, and preferably said peptide consists of 5 or 6 amino acid residues selected from relatively hydrophilic L-amino acids, for example, selected from the group consisting of Lys, Arg, Gly, Asn, Ala, Gln, Ser, Thr, Asp, Glu and Cys.

Specifically, as an especially preferred peptide consisting of the above-mentioned 5 or 6 amino acid residues, for example, a peptide consisting of 5 amino acid residues as shown by Lys-Arg-Gly-Cys-Gly- (SEQ ID NO: 1) may be mentioned. Further, as A, Lys-Arg-Arg-Ala-Gly- (SEQ ID NO: 2), Lys-Ala-Thr-Asp-Ala-Asn- (SEQ ID NO: 3), Lys-Ala-Thr-Asp-Cys-Asn- (SEQ ID NO: 4), and the like may be mentioned.

In the amino acid sequence of the formula (I), B represents a peptide consisting of 1 to 3 amino acid residues. Amino acids forming B are L-amino acids, and especially, a single amino acid or a peptide consisting of two amino acid residues, selected from L-amino acids having at their side chain a hydroxyl group or a mercapto group, for example, selected from the group consisting of Ser, Thr and Cys, is preferably mentioned. More specifically, as an especially preferred peptide consisting of the above-mentioned two amino acids, for example, a peptide represented by -Ser-Cys- may be mentioned. In addition, as B a single amino acid Ser may be mentioned.

The above-mentioned A and B can be used in any combination, provided that at least one of them contains a Cys residue.

In the formula (I), W$^1$ and W$^2$ represent, independently, Ser or Thr, and in the case where in a combination of A and B, A is Lys-Arg-Gly-Cys-Gly- (SEQ ID NO: 1) and B is -Ser-Cys- (referred as "preferred embodiment"), especially preferably W$^1$ is Thr and W$^2$ is Ser.

4

In the formula (I), X represents Lys or Arg, and where the combination of A and B is the above-mentioned "preferred embodiment", X is preferably Lys.

In the formula (I), $V^1$ and $V^2$ represent, independently, Ser, Arg, Thr, Tyr, Phe or Lys, and where the combination of A and B is the above-mentioned "preferred embodiment", $V^1$ and $V^2$ are preferably Phe or Lys, and the case wherein $V^1$ is Phe and $V^2$ is Lys is especially preferred.

In the formula (I), $Y^1$ and $Y^2$ represent, independently, Phe, Tyr or Trp, and where the combination of A and B is the above-mentioned "preferred embodiment", $Y^1$ and $Y^2$ are preferably Phe or Tyr, and the case wherein $Y^1$ is Tyr and $Y^2$ is Phe is especially preferred.

In the formula (I), Z represents Leu, Val, Ile, Pro, Phe or Ala, and where the combination of A and B is the above-mentioned "preferred embodiment", Z is preferably Leu.

A preferred embodiment of the present physiologically active peptides represented by the formula (I) is that wherein A is Lys-Arg-Gly-Cys-Gly-(SEQ ID NO: 1), B is -Ser-Cys, $W^1$ and $W^2$ are Ser or Thr, X is Lys or Arg, especially Lys, $V^1$ and $V^2$ are Phe or Lys, $Y^1$ and $Y^2$ are Phe or Tyr, and Z is Leu.

In the peptide portion $W^1$-X-$V^1$-Z-$W^2$-$Y^1$-$V^2$-$Y^2$ in the present peptide, any combination of the above-defined $W^1$, X, $V^1$, Z, $W^2$, $Y^1$, $V^2$ and $Y^2$ can be used, and as specific embodiments,

Thr-Lys-Phe-Leu-Ser-Tyr-Lys-Phe (SEQ ID NO: 5)
Thr-Lys-Thr-Val-Ser-Tyr-Thr-Phe (SEQ ID NO: 6)
Pro-Lys-Thr-Val-Ser-Tyr-Thr-Tyr (SEQ ID NO: 7)

are mentioned.

Accordingly, as an entire amino acid sequence of the present peptide, those wherein A is one of the amino acid sequences shown in SEQ ID NOs: 1 to 4, $W^1$ to $Y^2$ is one of the amino acid sequences shown in SEQ ID NOs: 5 to 7, B is Ser-Cys, or Ser are mentions. These peptides are exemplified as follow:

Lys-Arg-Gly-Cys-Gly-Thr-Lys-Phe-Leu-Ser-Tyr-Lys-Phe-Ser-Cys (SEQ ID NO: 8)
Lys-Arg-Gly-Cys-Gly-Thr-Lys-Phe-Leu-Ser-Tyr-Lys-Phe-Ser (SEQ ID NO: 9)
Lys-Arg-Gly-Cys-Gly-Thr-Lys-Thr-Val-Ser-Tyr-Thr-Phe-Ser-Cys (SEQ ID NO: 10)
Lys-Arg-Gly-Cys-Gly-Thr-Lys-Thr-Val-Ser-Tyr-Thr-Phe-Ser (SEQ ID NO: 11)
Lys-Arg-Gly-Cys-Gly-Pro-Lys-Thr-Val-Ser-Tyr-Thr-Phe-Tyr-Cys (SEQ ID NO: 12)
Lys-Arg-Gly-Cys-Gly-Pro-Lys-Thr-Val-Ser-Tyr-Thr-Tyr-Ser (SEQ ID NO: 13)
Lys-Arg-Arg-Ala-Gly-Thr-Lys-Phe-Leu-Ser-Tyr-Lys-Phe-Ser-Cys (SEQ ID NO: 14)
Lys-Arg-Arg-Ala-Gly-Thr-Lys-Thr-Val-Ser-Tyr-Thr-Phe-Ser-Cys (SEQ ID NO: 15)
Lys-Arg-Arg-Ala-Gly-Pro-Lys-Thr-Val-Ser-Tyr-Thr-Phe-Tyr-Cys (SEQ ID NO: 16)
Lys-Ala-Thr-Asp-Ala-Asn-Thr-Lys-Phe-Val-Ser-Tyr-Thr-Phe-Ser-Cys (SEQ ID NO: 17)
Lys-Ala-Thr-Asp-Ala-Asn-Thr-Lys-Thr-Val-Ser-Tyr-Thr-Phe-Ser-Cys (SEQ ID NO: 18)
Lys-Ala-Thr-Asp-Ala-Asn-Pro-Lys-Thr-Val-Ser-Tyr-Thr-Phe-Tyr-Cys (SEQ ID NO: 19)
Lys-Ala-Thr-Asp-Cys-Asn-Thr-Lys-Phe-Leu-Ser-Tyr-Lys-Phe-Ser-Cys (SEQ ID NO: 20)
Lys-Ala-Thr-Asp-Cys-Asn-Thr-Lys-Phe-Leu-Ser-Tyr-Lys-Phe-Ser (SEQ ID NO: 21)
Lys-Ala-Thr-Asp-Cys-Asn-Thr-Lys-Thr-Val-Ser-Tyr-Thr-Phe-Ser-Cys (SEQ ID NO: 22)
Lys-Ala-Thr-Asp-Cys-Asn-Thr-Lys-Thr-Val-Ser-Tyr-Thr-Phe-Ser (SEQ ID NO: 23)
Lys-Ala-Thr-Asp-Cys-Asn-Pro-Lys-Thr-Val-Ser-Tyr-Thr-Tyr-Ser-Cys (SEQ ID NO: 24)
Lys-Ala-Thr-Asp-Cys-Asn-Pro-Lys-Thr-Val-Ser-Tyr-Thr-Tyr-Ser (SEQ ID NO: 25)

The present physiologically active peptides represented by the formula (I) can be modified by replacement, deletion and/or insertion of one or more amino acid residues.

As a modified amino acid sequence corresponding to a peptide of other than type II PLA₂, a sequence portion similar to a physiologically active peptide defined by the above-mentioned formula (I) and found in an amino acid sequence of snake venom type II PLA₂ (Garel J. ran den Birgh et al., J. Cellular Biochemistry 39, p. 379 - 390 (1980)), for example, Lys-Ala-Thr-Asp-Cys-Asn-Pro-Lys-Thr-Val-Ser-Tyr-Thr-Tyr-Ser (SEQ ID NO: 25) can be mentioned. More specifically, those represented by the formula (I) wherein $W^1$ is Pro in place of Ser or Thr, and A, B, $W^2$, X, $V^1$, $V^2$, $Y^1$ and $Y^2$ are same as defined in the formula (I) can be mentioned.

Amino acid residues forming a physiologically active peptide shown by the formula (I) are defined as above, and these amino acids are not limited to L-amino acid as described above, and can include D-amino acids or $\beta$- or $\gamma$-amino acid rather than $\alpha$-amino acid, as far as a peptide formed therefrom has desired physiological properties such as an activity to inhibit binding of human type II phospholipase A with heparin, or an activity to inhibit human type II phospholipase A₂.

Functional groups of side chains of these amino acids, or an amino group and/or a carboxyl group of the N-terminal and/or the C-terminal of peptides represented by the formula (I) may be chemically modified or protected with known protecting groups, for example, may be esterified with, for example, methyl group, ethyl group, ethyl ester group, n-propyl ester group, isopropyl ester group, n-butyl ester group, sec-butyl

EP 0 687 685 A1

ester group, tert-butyl ester group, benzyl ester group, 2-cyanoethyl ester group etc.; alkylated with, for example, methyl group, ethyl group, n-propyl group, isopropyl group, n-butyl group, sec-butyl group, tert-butyl group, benzyl group, trityl group, carboxylmethyl group, aminocarboxy methyl group etc.; acylated with, for example, acetyl group, benzoyl group, benzyloxy carbonyl group, 9-fluorenylmethyloxycarbonyl (Fmoc), isobornyloxycarbonyl group etc.; or silylated with, for example, trimethylsilyl group, ethyl dimethyl silyl group, tert-butyl dimethyl silyl group, diisopropylmethyl silyl group etc.

According to the present invention, in the peptides explained in detail hereinbefore, at least one Cys residue contained in A and/or B is derivatized.

(1) According to the first embodiment, in a peptide containing at least one Cys residue in at least one of A and/or B, said at least one Cys is modified at the -SH group. This embodiment includes:

(1)-1 the case wherein only A contains Cys residue, and this Cys residue is modified;

(1)-2 the case wherein only B contains Cys residue, and this Cys residue is modified;

(1)-3 the case wherein both of A and B contain Cys residue, and only the Cys residue in A is modified; and

(1)-4 the case wherein both A and B contain Cys residue, and only the Cys in B is modified.

As groups modifying the Cys residue, there may be mentioned a carboxy lower alkyl group ($-C_nH_{2n}-COOH$) (n = 1 to 6), such as carboxymethyl group ($-CH_2-COOH$), substituted or non-substituted benzyl group, for example, lower alkylbenzyl group such as 4-methoxybenzyl group (Mob), acetoamide methyl group ($-CH_2NHCOCH_3$), 2-pyrydylsulfenyl group (SPy), 3-nitro-2-pyrydylsulfenyl group (Npys) and the like, and 4-methoxybenzyl group is especially preferred.

(2) According to the second embodiment, in one peptide containing at least one Cys residue in each of the A and B, a ring structure is formed by a sulfide bond between said two Cys residues.

(3) According to the third embodiment, in more than one peptide, a linear polymer is formed by one or more disulfide bonds formed between Cys residue contained in A or B of one peptide and Cys residue contained in A or B of another peptide. As polymers, dimers or trimers are preferred and dimers are especially preferred. The embodiments of the dimer include:

(3)-1 the case where a peptide (a) containing a Cys residue only in A and a peptide (b) containing a Cys residue only in A are linked by a disulfide bond formed between said Cys residues;

(3)-2 the case wherein a peptide (a) containing a Cys residue only in A and a peptide (b) containing a Cys residue only in B are linked by a disulfide bond formed between said Cys residues;

(3)-3 the case where a peptide (a) containing a Cys residue only in B and a peptide (b) containing a Cys residue only in B are linked by a disulfide bond formed between said Cys residues;

(3)-4 the case wherein a peptide (a) containing a Cys residue in each of A and B and a peptide (b) containing a Cys residue only in A are linked by a disulfide bond formed between said Cys residue in A of the peptide (a) and said Cys residue in A of the peptide (b);

(3)-5 the case wherein a peptide (a) containing a Cys residue in each of A and B and a peptide (b) containing a Cys residue only in A are linked by a disulfide bond formed between said Cys residue in B of the peptide (a) and said Cys residue in the peptide (b);

(3)-6 the case wherein a peptide (a) containing a Cys residue in each of A and B and a peptide (b) containing a Cys residue only in B are linked by a disulfide bond formed between said Cys residue in A of the peptide (a) and said Cys residue in B of the peptide (b);

(3)-7 the case wherein a peptide (a) containing a Cys residue in each of A and B and a peptide (b) containing a Cys residue only in B are linked by a disulfide bond formed between said Cys residue in B of the peptide (a) and said Cys residue in B of the peptide (b);

(3)-8 the case wherein a peptide (a) containing a Cys residue in each of A and B and a peptide (b) containing a Cys residure in each of A and B are linked by a disulfide bond formed between said Cys residue in A of the peptide (a) and said Cys residue in A of the peptide (b);

(3)-9 the case wherein a peptide (a) containing a Cys residue in each of A and B and a peptide (b) containing a Cys residue in each of A and B are linked by a disulfide bond formed between said Cys residue in A of the peptide (a) and said Cys in B of the peptide (b);

(3)-10 the case wherein a peptide (a) containing a Cys residue in each of A and B and a peptide containing a Cys residue in each of A and B are linked by a disulfide bond formed between said Cys residue in B of the peptide (a) and said Cys residue in B of the peptide (b).

In the above-mentioned polymers, amino acid sequences of more than one peptide may be same or different, and preferably same.

(4) According to the fourth embodiment, in more than one peptide wherein each of A and B contains at least one Cys residue, a polymeric ring structure has been formed by one or more disulfide bonds between a Cys residue contained in A or B of a peptide and a Cys residue contained in A or B of

6

another peptide.

The polymer is preferably dimer or trimer, and especially dimer is preferable. The embodiments of the dimer include;

(4)-1 the case wherein a peptide (a) containing Cys residue in each of A and B and peptide (b) containing Cys residue in each of A and B are linked by a disulfide bond formed between Cys residue in A of the peptide (a) and Cys residue in A of the peptide (b) and a disulfide bond formed between Cys residue in B of the peptide (a) and Cys residue in B of the peptide (b) so as to form a dimer ring structure; and

(4)-2 the case wherein a peptide (a) containing Cys residue in each of A and B and a peptide (b) containing Cys residue in each of A and B are linked by a disulfide bond formed between Cys residue in A of the peptide (a) and Cys residue in B of the peptide (b) and a disulfide bond formed between Cys residue in B of the peptide (a) and Cys residue in A of the peptide (b) to form a dimer ring structure.

(5) According to the fifth embodiment, in the structures (2) to (4) as described above, at least one Cys residue which does not form a disulfide bond is modified at its -SH group in a manner as described in (1). This embodiment includes the above-mentioned cases (3)-4, (3)-5, (3)-6, (3)-7, (3)-8, (3)-9 and (3)-10 wherein Cys residue which does not form a disulfide bond is modified.

Definite examples of the present invention include those shown in the following Tables 1 and 2. In these Tables, a structure of a peptide has the N-terminal at left side and the C-terminal at right side.

Table 1

| Name | Structure |
|---|---|
| Peptide (1) | K•R•G•C•G•T•K•F•L•S•Y•K•F•S•C<br>      └S—Mob                  └SH |
| Peptide (2) |      ┌S—Mob<br>K•R•G•C•G•T•K•F•L•S•Y•K•F•S•C<br>                              S<br>                              S<br>K•R•G•C•G•T•K•F•L•S•Y•K•F•S•C<br>      └S—Mob |
| Peptide (3) | K•R•G•C•G•T•K•F•L•S•Y•K•F•S•C<br>      └SH                 └S—Mob |
| Peptide (4) |                            ┌S—Mob<br>K•R•G•C•G•T•K•F•L•S•Y•K•F•S•C<br>          S<br>          S<br>K•R•G•C•G•T•K•F•L•S•Y•K•F•S•C<br>                  └S—Mob |
| Peptide (5) |      ┌S—Mob<br>K•R•G•C•G•T•K•F•L•S•Y•K•F•S•C<br>                              S<br>                              S<br>           K•R•G•C•G•T•K•F•L•S•Y•K•F•S•C<br>                                  └S—Mob |
| Peptide (6) | K•R•G•C•G•T•K•F•L•S•Y•K•F•S•C<br>      └S—Mob           └S—Mob |
| Peptide (7) | K•R•G•C•G•T•K•F•L•S•Y•K•F•S•C<br>      SH                  S<br>      SH                  S<br>K•R•G•C•G•T•K•F•L•S•Y•K•F•S•C |
| Peptide (8) | K•R•G•C•G•T•K•F•L•S•Y•K•F•S•C•SH<br>      S<br>      S<br>K•R•G•C•G•T•K•F•L•S•Y•K•F•S•C•SH |

```
                                      K•R•G•C•G•T•K•F•L•S•Y•K•F•S•C•SH
                                                    |
                                                    S
                                                    |
Peptide (9)                                         S
                        SH
                        |
                      K•R•G•C•G•T•K•F•L•S•Y•K•F•S•C
```

## Table 2

| Name | Structure |
|---|---|
| Peptide (10) | ```K•R•G•C•G•T•K•F•L•S•Y•K•F•S•C```<br>```     └─S—CH₂NHCOCH₃              └─SH``` |
| Peptide (11) | ```           ┌─S—CH₂NHCOCH₃```<br>```K•R•G•C•G•T•K•F•L•S•Y•K•F•S•C```<br>```                              S```<br>```                              S```<br>```K•R•G•C•G•T•K•F•L•S•Y•K•F•S•C```<br>```     └─S—CH₂NHCOCH₃``` |
| Peptide (12) | ```K•R•G•C•G•T•K•F•L•S•Y•K•F•S•C```<br>```     └─SH              └─S—CH₂NHCOCH₃``` |
| Peptide (13) | ```                           ┌─S—CH₂NHCOCH₃```<br>```K•R•G•C•G•T•K•F•L•S•Y•K•F•S•C```<br>```     S```<br>```     S```<br>```K•R•G•C•G•T•K•F•L•S•Y•K•F•S•C```<br>```                              └─S—CH₂NHCOCH₃``` |
| Peptide (14) | ```     ┌─S—CH₂NHCOCH₃```<br>```K•R•G•C•G•T•K•F•L•S•Y•K•F•S•C```<br>```                              S```<br>```                              S```<br>```          K•R•G•C•G•T•K•F•L•S•Y•K•F•S•C```<br>```                                 └─S—CH₂NHCOCH₃``` |
| Peptide (15) | ```K•R•G•C•G•T•K•F•L•S•Y•K•F•S•C```<br>```     └─S—CH₂NHCOCH₃       └─S—CH₂NHCOCH₃``` |

| | |
|---|---|
| Peptide (16) | K•R•G•C•G•T•K•F•L•S•Y•K•F•S•C<br>       \|                          \|<br>       S                         S<br>       \|                          \|<br>       S                         S<br>       \|                          \|<br>K•R•G•C•G•T•K•F•L•S•Y•K•F•S•C |
| Peptide (17) | S——————————————S<br> \|                                 \|<br>K•R•G•C•G•T•K•F•L•S•Y•K•F•S•C \|<br>       S————————S \|<br>       \|                      \|<br>K•R•G•C•G•T•K•F•L•S•Y•K•F•S•C |
| Peptide (18) | K•R•G•C•G•T•K•F•L•S•Y•K•F•S•C<br>       \|                        L—S—CH₂NHCOCH₃<br>       S–SPy $\quad$ $-$S–CH$_2$NHCOCH$_3$ |
| Peptide (19) | K•R•G•C•G•T•K•F•L•S•Y•K•F•S•C<br>       L—S–CH$_2$NHCOCH$_3$ $\qquad$ L—S–SPy |
| Peptide (20) | K•R•G•C•G•T•K•F•L•S•Y•K•F•S•C<br>       \|                          \|<br>       S————————————S |

Among the peptides described in the above-mentioned Tables 1 and 2, the peptides (2), (4), (17), (16), (11), (13) and (6) are preferred and as a protective group 4-methoxybenzyl group is preferred, and especially, the peptides (4), (17), (2) and (16) are preferable.

Alphabetical single letters in Tables 1 and 2 correspond to amino acid residues, and the symbol "•" between the alphabetical single letters represents a peptide bond. Mob represents a 4-methoxybenzyl group, and SPy represents a 2-pyridylsulfenyl group.

These physiologically active peptides of the present invention can be synthesized according to a conventional peptide synthesis process described, for example, in "Peptide Gosei No Kiso To Jikken (Nobuo Isumiya, Tetsuo Kato, Haruhiko Aoyagi, Michinori Waki: Maruzen K.K.) or "Solid Phase Peptide Synthesis, a Practical Approach" (LRL press), and can be purified.

In the case wherein side chains and/or peptide terminal of amino acid residues which form the present physiologically active peptide are chemically modified for example, esterified, or protected, they can be produced according to a conventional procedure ("Peptide Gosei No Kiso To Jikken" (Nobuo Izumiya, Ketsuo Kato, Haruhiko Aoyagi, Michinori Waki: Maruzen); "Tanpakushizu No Kagaku Shushoku" ⟨volume 1⟩ ⟨volume 2⟩ (Motonori Ono, Yuichi Kanaoka, Fumio Sakiyama, Hiroshi Maeda: (K.K.) Gakkai Shuppan Center), wherein a peptide is synthesized using chemically modified amino acids, alternatively a synthesized peptide is chemically modified.

The present peptides having an intermolecular disulfide bond can be obtained according to a conventional procedure, for example, method of "Peptide Gosei No Kiso To Jikken" (Nobuo Izumiya, Tetsuo Kato, Haruhiko Aoyagi, Michinori Waki: Maruzen (K.K.)), or E. Atherton, R.C. Sheppard, "Solid Phase Peptide Synthesis, a Practical Approach" (LRL press).

For example, the present peptide (2) is synthesized by a peptide synthesizer from starting amino acids forming a peptide (1) containing Cys protected with Mob, the resulting peptide (1) is dissolved in 10 mM ammonium acetate buffer (pH 8.0), and agitation is continued at room temperature for 3 days. In addition, if necessary, oxidative agents such as potassium fericyanate, hydrogen peroxide etc. can be added to accelerate the reaction. After that the peptide (2) can be purified and obtained.

In addition, peptide (7) can be obtained by adding m-cresol to the peptide (2), dropwise adding trifluoroacetic acid containing 1M trifluoromethanesulfonic acid and thioanisol thereon with ice cooling, adding anhydrous ether to form precipitates which are then centrifuged, removing supernatant, washing twice the precipitate with anhydrous ether, and purifying the precipitate by reverse phase HPLC.

Further, a desired peptide (16) can be obtained by dissolving the peptide (7) in 10 mM ammonium acetate buffer (pH 8.0), continuing agitation at room temperature for 3 days and purifying the product by reverse phase HPLC.

Peptide (4) can be obtained from peptide (3) according to the same procedure as that for obtaining peptide (2) from peptide (1). In addition, peptide (8) can be obtained from peptide (4) according to the same procedure as that for obtaining peptide (7) from peptide (2).

In addition, peptide (16) can be obtained from peptide (8) according to the same procedure as that for obtaining peptide (16) from peptide (7).

Peptide (1) and peptide (3) obtained in Examples 1 and 2 respectively are each dissolved in 10 mM ammonium acetate (pH 8.0) to a concentration of 1 mg/ml, and agitation is continued at room temperature for 3 days. In addition, if necessary, potassium ferricyanide, hydrogen peroxide etc. can be added thereto to accelerate the reaction. After that, the peptide is purified by reversed phase HPLC to obtain the desired peptide (5).

Peptide (9) is obtained by adding m-cresol to the peptide (5), dropwise adding trifluoroacetic acid containing 1M trifluoromethane sulfonic acid and thioanisol thereto with ice cooling, agitating the mixture for an hour, adding anhydrous ether to form precipitates which are centrifuged, removing supernatant, washing twice the precipitate with anhydrous ether, and purifying the precipitate by reverse phase HPLC.

Further, the peptide (9) is dissolved in 10 mM ammonium acetate buffer (pH 8.0), and agitation is continued at room temperature for 3 days. In addition, if necessary, an oxidant such as potassium ferricyanide, hydrogen peroxide etc. can be added to accelerate the reaction. After that, peptide (17) is purified and obtained.

Peptide (10) and peptide (12) obtained in Examples 3 and 4 respectively are each dissolved in 10 mM ammonium acetate (pH 8.0) to a concentration of 1 mg/ml, and agitation is continued at room temperature for 3 days. Alternatively, 2,2'-dithiobispyridine/acetic acid solution is added to peptide (10) or peptide (12), and the mixture is stirred for 30 minutes to obtain peptide (18) or peptide (19) respectively. Peptide (18) or (19) and peptide (10) or (12) are dissolved by an equivalent amount into 1N acetic acid aqueous solution, and the mixture is addicted to pH 6.5 and stirred for 30 minutes. After that the desired peptide (14) is purified by reverse phase HPLC and obtained.

The peptide (14) is dissolved in 90% methanol, 6N hydrochloric acid is added thereto, and 20 mM iodine/methanol solution is added to the reaction mixture with ice cooling, which is stirred for an hour. Alternatively, the peptide (14) is dissolved in 2N hydrochloric acid aqueous solution, and 1 mg/ml iodine/35% acetic acid to the reaction mixture, which is then stirred at room temperature for 30 minutes. After that, an ascorbic acid aqueous solution is added to the reaction mixture until the color of the reaction mixture disappears to decompose an excess amount of iodine, and reverse phase HPLC is carried out to obtain 2 mg of peptide (17).

Physiologically active peptides of the present invention represented by the formula (1) may be converted to corresponding salts or hydrate salts thereof according to known procedures. The salts are preferably nontoxic and water soluble. Suitable salts are inorganic salts such as hydrochloride, hydrobromide, sulfate, phosphate, nitrate etc., and organic acids such as acetate, trifluoroacetate, lactate, tartanate, oxalate, fumarate, maleate, citrate, benzoate, methanosulfonate, benzenesulfonate, toluenesulfonate, isothionate, glucronate, gluconate etc.

Physiological peptides of the present invention have low toxicity, and are safe enough to use them as pharmaceuticals.

The present physiologically active peptides have activities to inhibit binding of type II $PLA_2$ and heparin, and to inhibit enzyme activity of type II $PLA_2$ in the presence of heparin. Therefore, these peptides are useful for prophylactic and/or therapeutic treatments of various inflammatory diseases (for example, chronic rheumatic arthritis, organ distinction), asthma, allergic diseases, disseminated intravascular coagulation syndrome (DIC), vessel disorders (mycocardiac infarction, cerebral infarction), restenosis, ulcer, sepsis, ARDS, nephritis, pancreatitis, as well as increase of severity of pancreatitis and multi-organic disorders.

The present physiologically active peptides, nontoxic salts thereof or hydrate salts thereof are used by administrating systemically or topically, orally or parenterally. The administration dose depends on the age, symptoms, therapeutic effects, administration route, treatment schedule etc., and is usually 1 mg to 1000 mg per head per time, one to several times per day, for oral administration; or 1 mg to 100 mg per day per time, for parental administration (preferably intravenous administration); alternatively they are intravenously administrated continuously for 1 to 24 hours per day.

As described above, since administration dose varies depending on various conditions, the dose may be less than or more than the above-mentioned dose range. The present physiologically active peptides may be parenterally administrated as injections, topical drugs or suppositories etc. Compositions for oral administration include tablets, dragees, capsules, powders, particles etc. Capsules include hard capsules and soft capsules. Liquid compositions for oral administration include emulsion, solution, suspension, syrup, elixirs etc., and may contain conventional inactive diluting agents (water, ethanol etc.).

Injective agents for parenteral administration include sterilized aqueous or unaqueous solutions, suspension, emulsion etc. Other compositions for parenteral administration contain one or more active substances, and include external medicines such as external solution, ointment, liniment, as well as suppository and pessary for rectal administration.

As carriers and excipients for formulation, solid or liquid nontoxic medical substances are mentioned. They are exemplified by lactose, magnesium stearate, starch, talc, gelatin, agar, pectin, gum arabic, olive oil, sesame oil, cacao butter, ethyleneglycol and the like.

EXAMPLES

Next, the present invention is explained more in detail by reference examples and examples. In the examples, the references to, for example, peptide (1) etc. correspond to those described in Tables 1 and 2.

Reference Example 1

Preparation of human type II (non-pancreatic) Phospholipase $A_2$ ($PLA_2$)

Human type II $PLA_2$ was prepared by recombinant technique according to a report of J.J. Seilhamer et al., (J. Biol. Chem. Vol. 264, p. 5335 (1989)).

A DNA fragment for human type II $PLA_2$ amplified from a cDNA library by PCR method was introduced to a baculovirus recombinant vector pAcTJI1 (expression plasmid constructed on the basis of commercially available pVL1393 and pACYM1), which was then transfected to insect cells SF-9 together with vaculovirus (AcNPV) DNA, to obtain a recombinant virus.

Three days after infection with the recombinant virus, a culture supernatant of the SF 9 cells was harvested, and passed through a heparin Sepharose column or sulfated cellulofine column (Seikagaku Kogyo).

Reference Example 2

Biotination of human type II (non-pancreatic) $PLA_2$

500 $\mu$g of human type II (non-pancreatic) purified in Reference Example 1 was reacted with 2 ml of a solution of 5 mM sulfosuccinyl 6-(biotinamide hexose) in 0.1M $NaHCO_3$ at a room temperature for one hour, and the product was purified by a heparin Sepharose column to obtain biotinated human type II $PLA_2$.

Reference Example 3

Preparation of heparin-bound plate

50 $\mu$l of a phosphate buffered saline (PBS) containing 50 $\mu$g/ml poly-L-lysine was added to each well of a 96-well ELISA plate, and after incubation at room temperature for an hour, each well was twice washed with PBS, 50 $\mu$l of PBS solution containing 20 $\mu$g/ml heparin was added to each well, and a reaction was further carried out at a room temperature for an hour. Each well was twice washed with PBS, 200 $\mu$l of PBS solution containing 30 mg/ml bovine serum albumin (BSA) was added to each well, and after reaction at 37°C for an hour, the plate was stored at 4°C.

Example 1

## Synthesis and purification of Lys-Arg-Gly-Cys(Mob)-Gly-Thr-Lys-Phe-Leu-Ser-Tyr-Lys-Phe-Ser-Cys (peptide (1)) and homodimer thereof (peptide (2))

Peptide (1)

A   =   Lys-Arg-Gly-Cys(Mob)-Gly-
B   =   -Ser-Cys
$W^1$ =   Thr, X = Lys, $V^1$ = Phe, Z = Leu, $W^2$ = Ser,

$Y^1$ = Tyr, $V^2$ = Lys, $Y^2$ = Phe

Mob: 4-methoxybenzyl-

1) Starting from amino acids comprising a monomer peptide having the above-mentioned amino acid sequence, the peptide (1) was synthesized by a Fmoc method using a 431 type peptide synthesizer of Applied Biosystems. Deprotection was carried out by a TFA cleavage method. Purification was carried out by reverse phase HPLC (column: YMC-Pack R & D ODS S-5 12OA, 20 × 250 mm, flow rate 15.0 ml/min., mobile phase: 0.1% trifluoroacetic acid, 0 to 50% acetonitril concentration gradient, 60 minutes).

2) Peptide (2)

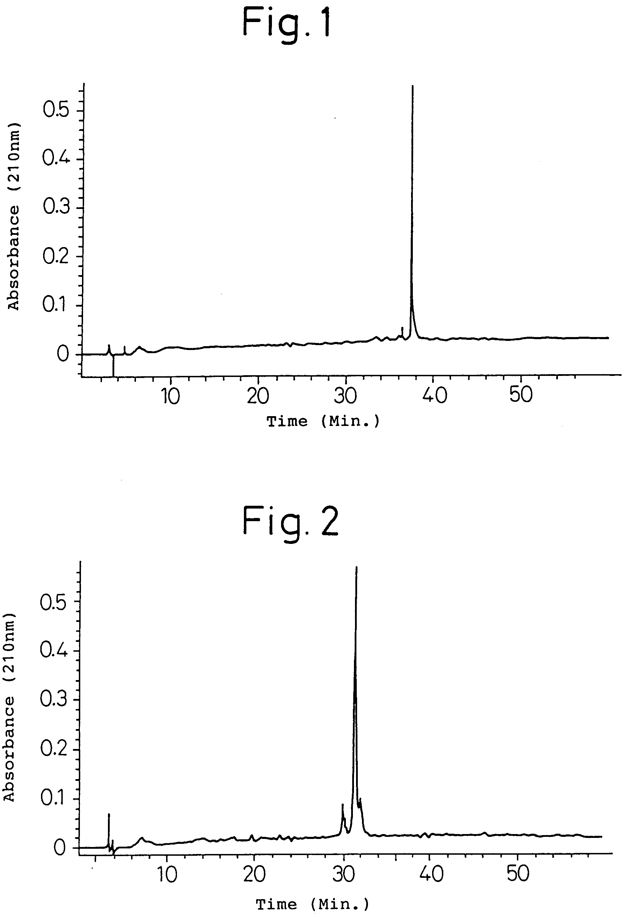

10 mg of the peptide (1) obtained in the above-mentioned step 1 was dissolved in 10 ml of 10 mM ammonium acetate buffer (pH 8.0), and after stirring at room temperature for 3 days, purification was carried out according to the same procedure as for the peptide (1) to obtain 5 mg of the desired peptide (2). A result of the HPLC (absorption at 210 nm) is shown in Fig. 1.

Note that the peptide thus obtained was confirmed to have the above-mentioned amino acid sequence by a peptide sequencer (Applied Biosystems).

3) Peptide (6)

Peptide (6) was synthesized using as starting material amino acids comprising the peptide (6) according to the some procedure as in 1. The resulting peptide was confirmed to have the expected amino acid sequence by a peptide sequencer.

Example 2

## Synthesis and purification of Lys-Arg-Gly-Cys-Gly-Thr-Lys-Phe-Leu-Ser-Tyr-Lys-Phe-Ser-Cys(Mob) (peptide (3) and homodimer thereof (peptide (4))

Peptide (3)

A = Lys-Arg-Gly-Cys-Gly-

B = -Ser-Cys(Mob)

$W^1$ = Thr, X = Lys, $V^1$ = Phe, Z = Leu, $W^2$ = Ser,

$Y^1$ = Tyr, $V^2$ = Lys, $Y^2$ = Phe

Mob: 4-methoxybenzyl-

1) Starting from amino acids comprising a monomer peptide (3) having the above-mentioned amino acid sequence, the peptide (3) was synthesized by a Fmoc method using a 431 type peptide synthesizer of Applied Biosystems. Deprotection was carried out by a TFA cleavage method. Purification was carried out by reverse phase HPLC (column: YMC-Pack R & D ODS S-5 120A, 20 × 250 mm, flow rate 15.0 ml/min., mobile phase: 0.1% trifluoroacetic acid, 0 to 50% acetonitryle concentration gradient, 60 minutes) to obtain 10 mg of the peptide (3).

Note that the peptide thus obtained was confirmed to have the above-mentioned amino acid sequence by a peptide sequencer (Applied Biosystems).

2) Peptide (4)

100 mg of the peptide (3) obtained in the above-mentioned step 1 was dissolved in 10 ml of 50 mM ammonium acetate buffer (pH 8.0), and after adding 60 $\mu$l of 0.3% hydrogen peroxide aqueous solution with ice cooling and continuing stirring for 30 minutes, purification was carried out by reversed phase HPLC according to the same procedure as for the peptide (3) to obtain 4 mg of the desired peptide (4).

Note that the peptide thus obtained was confirmed to have the above-mentioned amino acid sequence by a peptide sequencer (Applied Biosystems).

Example 3

## Synthesis and purification of Lys-Arg-Gly-Cys(Acm)-Gly-Thr-Lys-Phe-Leu-Ser-Tyr-Lys-Phe-Ser-Cys (peptide (10) and homodimers thereof (peptides (11) and (16))

Peptide (10)

A = Lys-Arg-Gly-Cys(Acm)-Gly-
B = -Ser-Cys
$W^1$ = Thr, X = Lys, $V^1$ = Phe, Z = Leu, $W^2$ = Ser,
$Y^1$ = Tyr, $V^2$ = Lys, $Y^2$ = Phe
Acm: acetamidemethyl-

1) Starting from amino acids comprising a monomer peptide (10) having the above-mentioned amino acid sequence, the peptide (10) was synthesized by a Fmoc method using a 431 type peptide synthesizer of Applied Biosynthesis. Deprotection was carried out by a TFA cleavage method. Purification was carried out by reverse phase HPLC (column: YMC-Pack R & D ODS S-5 12OA, 20 × 250 mm, flow rate 15.0 ml/min., mobile phase: 0.1% trifluoroacetic acid, 0 to 50% acetonitryle concentration gradient, 60 minutes).

2) Peptide (11)

10 mg of the peptide (10) obtained in the above-mentioned step 1 was dissolved in 10 ml of 10 mM ammonium acetate buffer (pH 8.0), and after continuously stirring at room temperature for 3 days, purification was carried out by reverse phase HPLC according to the same procedure as for the peptide (1) to obtain 5 mg of the desired peptide (11). A result of the HPLC (absorption at 210 nm) is shown in Fig. 2.

3) Peptide (16)

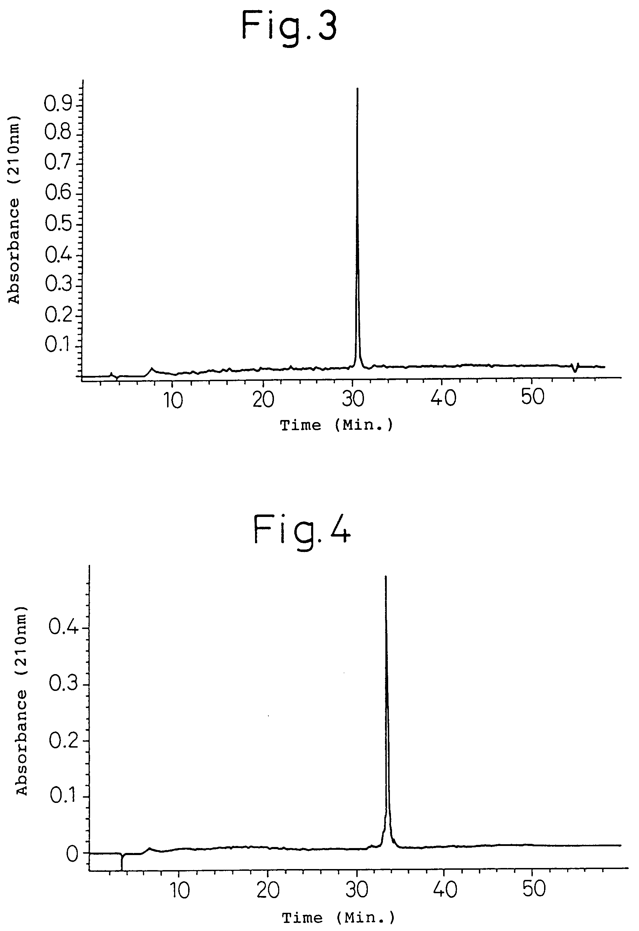

5 mg of the peptide (11) obtained in the above-mentioned step 2 was dissolved in 50 ml of 2N hydrochloric acid aqueous solution, and to the solution was added 5.3 ml of 1 mg/ml iodine/35% acetic acid solution, and the mixture was stirred at room temperature for 30 minutes. After that, 4 mg/ml ascorbic acid aqueous solution was added until the color of the reaction mixture disappeared, to consume the excess amount of iodine, and purification was carried out by reverse phase HPLC according to the same procedure as for the peptide (1) to obtain 4.5 mg of the desired peptide (16). A result of the HPLC (absorption at 210 nm) is shown in Fig. 3.

Note the peptide thus obtained was confirmed to have the above-mentioned amino acid sequence by a peptide sequencer (Applied Biosystems). In addition, the peptide (16) thus obtained was confirmed to have a molecular weight of about 3446 by ion bombardment mass spectrometry.

Example 4

## Synthesis and purification of Lys-Arg-Gly-Cys-Gly-Thr-Lys-Phe-Leu-Ser-Tyr-Lys-Phe-Ser-Cys(Acm) (peptide (12) and homodimers (peptides (13) and (16))

Peptide (12)

A = Lys-Arg-Gly-Cys-Gly-
B = -Ser-Cys(Acm)
$W^1$ = Thr, X = Lys, $V^1$ = Phe, Z = Leu, $W^2$ = Ser,

14

Y¹ =    Tyr, V² = Lys, Y² = Phe

Acm:    acetamidemethyl-

1) Starting from amino acids comprising a monomer peptide (12) having the above-mentioned amino acid sequence, the peptide (12) was synthesized by a Fmoc method using a 431 type peptide synthesizer of Applied Biosystems. Deprotection was carried out by TFA cleavage. Purification was carried out by reverse phase HPLC (column: YMC-Pack R & D ODS S-5 120A, 20 × 250 mm, flow rate 15.0 ml/min., mobile phase: 0.1% trifluoroacetic acid, 0 to 50% acetonitryle concentration gradient, 60 minutes).

2) Peptide (13)

10 mg of the peptide (12) obtained in the above-mentioned 1) was dissolved in 10 ml of 10 mM ammonium acetate (pH 8.0), and after continuously stirring at room temperature for 3 days, purification was carried out by reversed phase HPLC according to the same procedure as for the peptide (1) to obtain 5 mg of the desired peptide (13). A result of the HPLC (absorption at 210 nm) is shown in Fig. 4.

3) Peptide (16)

5 mg of the peptide (13) obtained in the above-mentioned 2) was dissolved in 50 ml of 2N hydrochloric acid aqueous solution, to the solution was added 5.3 ml of 1 mg/ml iodine/35% acetic acid solution, and the mixture was stirred at room temperature for 30 minutes. After that, 4 mg/ml ascorbic acid aqueous solution was added until the color of the reaction mixture disappeared, to consume the excess amount of iodine, and purification was carried out by reverse phase HPLC according to the same procedure as for the peptide (1) to obtain 4.5 mg of the desired peptide (16).

Note that the peptide thus obtained was confirmed to have the above-mentioned amino acid sequence by a peptide sequencer (Applied Biosystems). In addition, the peptide (16) thus obtained was confirmed to have a molecular weight of about 3446 by ion spray mass spectrometry.

Example 5

## Synthesis and purification of Lys-Arg-Gly-Cys-Gly-Thr-Lys-Phe-Leu-Ser-Tyr-Lys-Phe-Ser-Cys (peptide (21))

A =    Lys-Arg-Gly-Cys-Gly-

B =    -Ser-Cys

W¹ =   Thr, X = Lys, V¹ = Phe, Z = Leu, W² = Ser,

Y¹ =   Tyr, V² = Lys, Y² = Phe

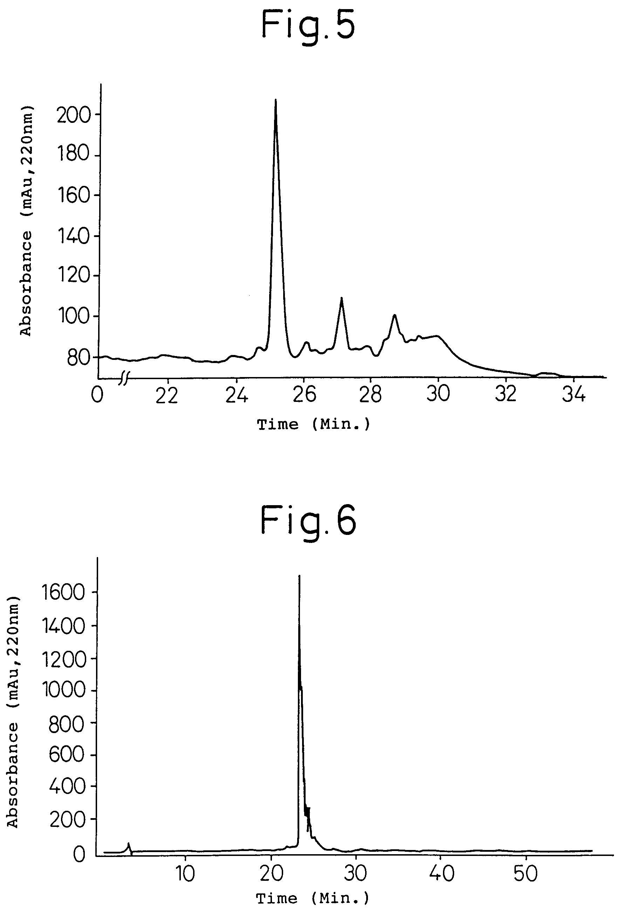

Starting from amino acids comprising the above-mentioned peptide (21), the peptide (21) was synthesized by a Fmoc method using a 431 type peptide synthesizer of Applied Biosystems. Deprotection was carried out by a TMS Br cleavage method. Purification was carried out by reverse phase HPLC (column: Vydac Protein C4, 20 × 25 mm, flow rate 25 ml/min., mobile phase: 0.1% trifluoroacetic acid, 5 to 55% acetonitryle concentration gradient, 60 minutes). A result of the HPLC (absorption at 210 nm) is shown in Fig. 5. As can be seen from Fig. 5, the desired peptide (21) was obtained at a retention time of 25.2 to 25.4 minutes. Reaction yield was 45%. The amino acid sequence of the peptide (21) thus obtained was confirmed by a gas phase protein sequencer 477 of Applied Biosystems.

This peptide (21) is converted to a biologically active peptide of the present invention by modification of Cys residues in A and/or B, and/or formation of disulfide bond.

Example 6

## Synthesis and purification of heterodimer of Lys-Arg-Gly-Cys-Gly-Thr-Lys-Phe-Leu-Ser-Tyr-Lys-Phe-Ser-Cys (peptide (17))

(Peptide (17)

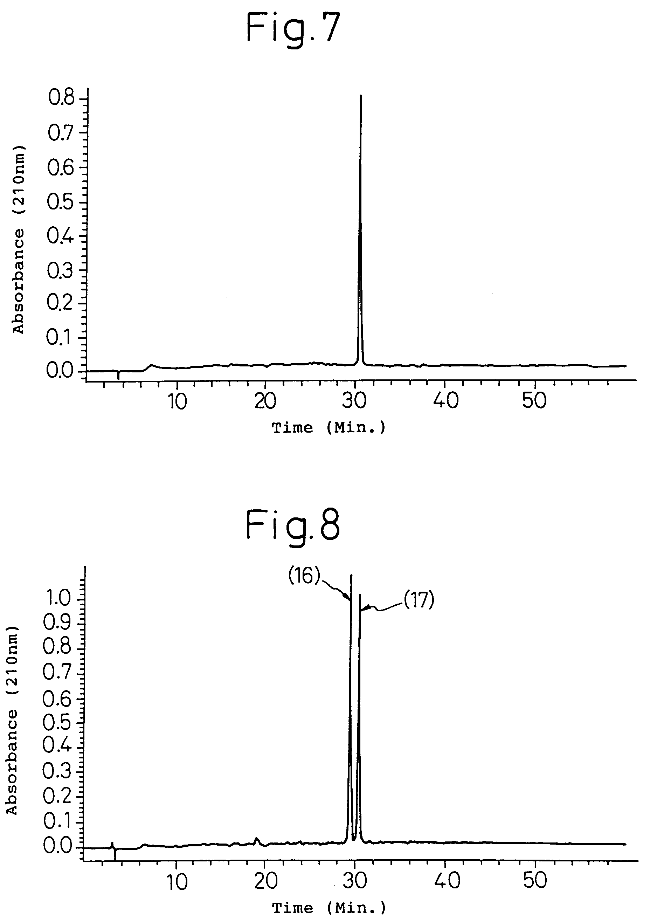

10 mg of the peptide (21) obtained in Example 5 was dissolved in 10 ml of 10 mM ammonium acetate (pH 6.0), and after stirring at room temperature for an hour, purification was carried out by reverse phase HPLC (column: YMC-Pack R & D ODS S-5 120A, 20 $\times$ 250 mm, flow rate 15.0 ml/min., mobile phase: 0.1% trifluoroacetic acid, 0 to 50% acetonitrile concentration gradient, 60 minutes) to obtain 5 mg of the desired peptide (17). A result of this HPLC (absorption at 120 nm) is shown in Fig. 7.

In addition, to confirm that the peptide (17) thus obtained has the expected structure, the peptide (17) and the peptide (16), which has the same molecular weight as the peptide (17) wherein peptide chains are reversed, were double-injected for HPLC. A result is shown in Fig. 8.

Note that the peptide obtained was confirmed to have the above-mentioned amino acid sequence by a peptide sequencer (Applied Biosynthesis). In addition, the peptide (17) was confirmed to have a molecular weight of about 3446 by ion spray mass spectrometry.

Example 7

## Synthesis and purification of Lys-Arg-Gly-Cys-Gly-Thr-Lys-Phe-Leu-Ser-Tyr-Lys-Phe-Ser-Cys

(Peptide (20))

A = Lys-Arg-Gly-Cys-Gly-
B = -Ser-Cys
$W^1$ = Thr, X = Lys, $V^1$ = Phe, Z = Leu, $W^2$ = Ser,
$Y^1$ = Tyr, $V^2$ = Lys, $Y^2$ = Phe

3 mg of the peptide obtained in Example 5 was dissolved in 1L of ammonium acetate (pH 8.0), and after air oxidation at room temperature, the desired peptide was isolated by reverse phase HPLC to obtain 2.5 mg of the desired peptide (20).

Example 8

## Synthesis and purification of Lys-Arg-Gly-Cys(CM)-Gly-Thr-Lys-Phe-Leu-Ser-Tyr-Lys-Phe-Ser-Cys(CM) (peptide (22)) (CM = S-carboxymethyl)

A = Lys-Arg-Gly-Cys(CM)-Gly-
B = -Ser-Cys(CM)
$W^1$ = Thr, X = Lys, $V^1$ = Phe, Z = Leu, $W^2$ = Ser,
$Y^1$ = Tyr, $V^2$ = Lys, $Y^2$ = Phe

5 mg of the peptide (21) obtained in Example 5 was reacted with 2-iodoacetic acid in the presence of guanidine hydrochloride to introduce a carboxymethyl group into the $\beta$-thiol group of the Cys. Purification was carried out by reverse phase HPLC according to the same procedure as for the peptide (21) to obtain about 3.8 mg of the desired peptide (22). A result of the HPLC (absorption at 210 nm) is shown in Fig. 6. As

EP 0 687 685 A1

can be seen from Fig. 6, the desired peptide (22) was obtained at a retention time of 23.2 to 23.8 minutes. In addition, according to the same procedure as for the peptide (21), the amino acid sequence of the peptide (22) was analyzed by a protein sequencer, to confirm the presence of S-carboxymethyl cysteine.

Example 9

### Synthesis and purification of Lys-Ala-Thr-Asp-Ala-Asn-Thr-Lys-Thr-Val-Ser-Tyr-Thr-Tyr-Ser-Cys (peptide (23))

A = Lys-Ala-Thr-Asp-Ala-Asn-
B = -Ser-Cys
$W^1$ = Thr, X = Lys, $V^1$ = Thr, Z = Val, $W^2$ = Ser,
$Y^1$ = Tyr, $V^2$ = Thr, $Y^2$ = Phe

The peptide (23) was synthesized and purified according to the same procedure as in Example 5, and its amino acid sequence was confirmed.

The peptide (23) is converted to a linear dimer by modification of the Cys residue in B, or formation of a disulfide bond between two molecules.

Example 10

### Synthesis and purification of Lys-Ala-Thr-Asp-Cys-Asn-Pro-Lys-Thr-Val-Ser-Tyr-Thr-Tyr-Ser (peptide (24))

A = Lys-Ala-Thr-Asp-Cys-Asn-
B = -Ser
$W^1$ = Pro, X = Lys, $V^1$ = Thr, Z = Val, $W^2$ = Ser,
$Y^1$ = Tyr, $V^2$ = Thr, $Y^2$ = Tyr

The peptide (24) was synthesized and purified according to the same procedure as described in Example 5, and its amino acid sequence was confirmed.

The peptide (24) is converted to a linear dimer by modification of the Cys residue in A or formation of a disulfide bond between two molecules.

Reference Example 4

### Synthesis and purification of Lys-Arg-Gly-Ala-Gly-Thr-Lys-Phe-Leu-Ser-Tyr-Lys-Phe-Ser (peptide (25))

A = Lys-Arg-Gly-Ala-Gly-
B = -Ser
$W^1$ = Thr, X = Lys, $V^1$ = Phe, Z = Leu, $W^2$ = Ser,
$Y^1$ = Tyr, $V^2$ = Lys, $Y^2$ = Phe

The peptide (25) was synthesized and purified according to the same procedure as in Example 5, and its amino acid sequence was confirmed.

17

Reference Example 5

Synthesis and purification of Gly-Thr-Lys-Phe-Leu-Ser-Tyr-Lys-Phe-Ser (peptide (26))

A    =    Gly-
B    =    -Ser
$W^1$ =    Thr, X = Lys, $V^1$ = Phe, Z = Leu, $W^2$ = Ser,
$Y^1$ =    Tyr, $V^2$ = Lys, $Y^2$ = Phe

The peptide (26) was synthesized and purified according to the same procedure as in Example 5, and its amino acid sequence was confirmed.

Reference Example 6

Synthesis and purification of Asn-Thr-Lys-Thr-Val-Ser-Tyr-Thr-Tyr-Ser (peptide (27))

A    =    Asn-
B    =    -Ser
$W^1$ =    Thr, X = Lys, $V^1$ = Thr, Z = Val, $W^2$ = Ser,
$Y^1$ =    Tyr, $V^2$ = Thr, $Y^2$ = Phe

The peptide (27) was synthesized and purified according to the same procedure as in Example 5, and its amino acid sequence was confirmed.

Reference Example 7

Synthesis and purification of Asn-Pro-Lys-Thr-Val-Ser-Tyr-Thr-Tyr-Ser (peptide (28))

The peptide (28) was obtained by replacement of $W^1$(Thr) in the peptide (27) with Pro.
The peptide (28) was synthesized and purified according to the same procedure as in Example 7.

Example 11

Measurement of inhibition of binding between heparin and human II-type $PLA_2$

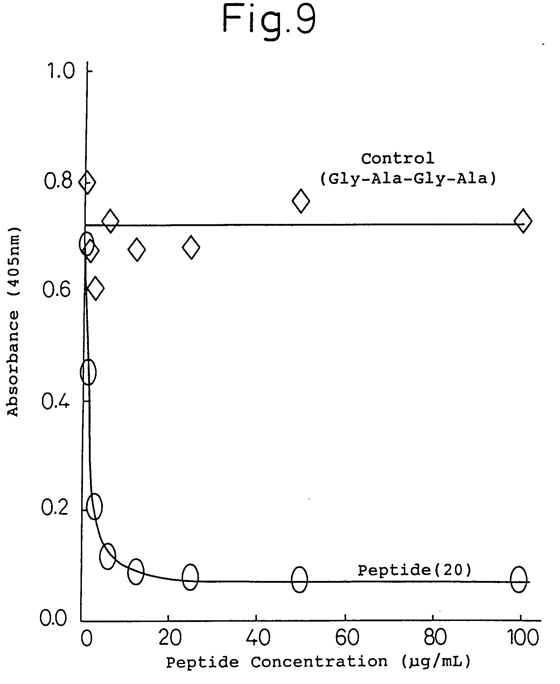

To each well of a peparin-bound plate prepared in Reference Example 3, 50 $\mu$l of a solution containing a peptide obtained in Examples 1 to 10 was added. After incubating at room temperature for 45 minutes, 50 $\mu$g/ml biotinated human II-type $PLA_2$ obtained in Example 2 was added thereon, and reaction was further carried out at room temperature for an hour. Each well was washed three times with PBS containing 0.05% Tween 20, 50 $\mu$l of a 3% BSA/PBS solution containing alkaline phosphatase-conjugated streptoavidin (Taga, $\times$2000 dilution) was added thereon, and a reaction was carried out at a room temperature for an hour. Each well was washed three times with PBS containing 0.05% Tween 20, 100 $\mu$l of a 1 mM p-nitrophenylphosphate/1M ethanolamine aqueous solution (pH 9.8) was added thereon, and a reaction was carried out at room temperature for an hour. Absorption of reaction mixture was measured at 405 nm using a microplate reader (Molecular Device Inc). A result is shown in Fig. 9, and $IC_{50}$ ($\mu$M) was also obtained.

The results are shown in Table 3. As can be seen from the Table, the peptides (1), (2), (4), (6), (11), (13), (14), (16), (19), and (20) have strong inhibitory activity to binding of human type II $PLA_2$ to heparin.

## Table 3

| Name | Structure | Inhibition: $IC_{50}$ ($\mu M$) |
|---|---|---|
| Peptide (1) | K•R•G•C•G•T•K•F•L•S•Y•K•F•S•C<br>　　　└─S—Mob　　　　　　　└─SH | 1.4 |
| Peptide (2) | 　　　┌─S—Mob<br>K•R•G•C•G•T•K•F•L•S•Y•K•F•S•C<br>　　　　　　　　　　　　　　S<br>　　　　　　　　　　　　　　S<br>K•R•G•C•G•T•K•F•L•S•Y•K•F•S•C<br>　　　└─S—Mob | 0.13 |
| Peptide (3) | K•R•G•C•G•T•K•F•L•S•Y•K•F•S•C<br>　　　└─SH　　　　　　　└─S—Mob | 2.7 |
| Peptide (4) | 　　　　　　　　　　　　┌─S—Mob<br>K•R•G•C•G•T•K•F•L•S•Y•K•F•S•C<br>　　　S<br>　　　S<br>K•R•G•C•G•T•K•F•L•S•Y•K•F•S•C<br>　　　　　　　　　　　　└─S—Mob | 0.68 |
| Peptide (6) | K•R•G•C•G•T•K•F•L•S•Y•K•F•S•C<br>　　　└─S—Mob　　　　　└─S—Mob | 3.2 |
| Peptide (11) | 　　　┌─S—CH₂NHCOCH₃<br>K•R•G•C•G•T•K•F•L•S•Y•K•F•S•C<br>　　　　　　　　　　　　　　S<br>　　　　　　　　　　　　　　S<br>K•R•G•C•G•T•K•F•L•S•Y•K•F•S•C<br>　　　└─S—CH₂NHCOCH₃ | 0.75 |

| | | |
|---|---|---|
| Peptide (13) | K•R•G•C•G•T•K•F•L•S•Y•K•F•S•C ⌐S—CH₂NHCOCH₃<br>(disulfide bridges)<br>K•R•G•C•G•T•K•F•L•S•Y•K•F•S•C ⌐S—CH₂NHCOCH₃ | 1.1 |
| Peptide (16) | K•R•G•C•G•T•K•F•L•S•Y•K•F•S•C<br>(S—S bridges)<br>K•R•G•C•G•T•K•F•L•S•Y•K•F•S•C | 0.51 |
| Peptide (17) | K•R•G•C•G•T•K•F•L•S•Y•K•F•S•C<br>(S—S bridges)<br>K•R•G•C•G•T•K•F•L•S•Y•K•F•S•C | 0.07 |
| Peptide (20) | K•R•G•C•G•T•K•F•L•S•Y•K•F•S•C<br>S————————S | 0.03 |

Example 12

Measurement of enzyme inhibition of human type II PLA₂ in the presence of heparin

Enzyme inhibitory activity of the peptide (16) obtained in Example 1 in the presence of heparin of bovine small intestine origin (Sigma, catalog No. H3393) (10 μg/ml) was measured by the method of Dole (Natori, Y et al., J. Biochem., 93, 631 - 637 (1983)) using a phospholipid (1-acyl-2-arachidonyl-L-3-phosphatidylethanolamine) containing $C^{13}$-radiolabeled arachidonic acid, and by a method using a membrane fragment (NEN) prepared by incorporating radiolabeled arachidonic acid to membrane phospholipide of E. coli (Ruth M. Kramer et al., J. Biol Chem., 264, 5768 - 5775 (1989)). As a result, in both of the measurement systems, the peptide (16) was confirmed to have an inhibitory activity to type II PLA₂ enzyme (in Table, shown as PLA₂ enzyme). In addition, the peptides (2), (11), (13), (19) and (20) also have an inhibitory activity to type II PLA₂ enzyme. The results are shown in Table 4.

Table 4

| Assessment of enzyme inhibitory activity using radiolabeled synthetic phospholipid | | | | | |
|---|---|---|---|---|---|
| Peptide | Peptide concentration ($\mu$g/ml) | Heparin concentration ($\mu$g/ml) | PLA$_2$ enzyme concentration (ng/ml) | PLA$_2$ enzyme activity (%) | Inhibition (%) |
| (16) | 0 | 0 | 200 | 100 | 0 |
| | 5 | 50 | 200 | 41 | 59 |
| | 10 | 50 | 200 | 28 | 72 |
| | 50 | 50 | 200 | 18 | 82 |
| | 100 | 50 | 200 | 10 | 90 |
| (2) | 0 | 50 | 200 | 100 | 0 |
| | 5 | 50 | 200 | 75 | 25 |
| | 10 | 50 | 200 | 48 | 52 |
| | 50 | 50 | 200 | 19 | 81 |
| | 100 | 50 | 200 | 11 | 89 |
| (11) | 5 | 50 | 200 | 57 | 43 |
| | 10 | 50 | 200 | 37 | 63 |
| | 50 | 50 | 200 | 22 | 78 |
| | 100 | 50 | 200 | 18 | 82 |
| (13) | 5 | 50 | 200 | 58 | 42 |
| | 10 | 50 | 200 | 38 | 62 |
| | 50 | 50 | 200 | 19 | 81 |
| | 100 | 50 | 200 | 14 | 86 |
| (17) | 0 | 0 | 200 | 100 | 0 |
| | 1 | 10 | 200 | 31 | 69 |
| | 5 | 10 | 200 | 9 | 91 |
| | 10 | 10 | 200 | 6 | 94 |
| (20) | 0 | 50 | 200 | 100 | 0 |
| | 1 | 50 | 200 | 90 | 10 |
| | 5 | 50 | 200 | 41 | 59 |
| | 10 | 50 | 200 | 28 | 72 |
| | 50 | 50 | 200 | 18 | 82 |
| | 100 | 50 | 200 | 10 | 90 |

Table 5

| Assessment of enzyme inhibitory activity using radioactive phospholipid of E. coli origin | | | | |
|---|---|---|---|---|
| Peptide (16) concentration ($\mu$g/ml) | Heparin concentration ($\mu$g/ml) | PLA$_2$ enzyme concentration (ng/ml) | PLA$_2$ enzyme activity (%) | Inhibition (%) |
| 0 | 0 | 10 | 100 | 0 |
| 0 | 10 | 10 | 64 | 36 |
| 1 | 10 | 10 | 12 | 88 |
| 5 | 10 | 10 | 3 | 97 |
| 10 | 10 | 10 | 2 | 98 |

Example 13

Effects of peptide of the present invention on mouse endotoxin shock model

1) A mouse endotoxin shock model was constructed according to the method of Galanos et al. (Proc. Natl. Acad. Sci, USA 76, 5943 - 5943, 1979). Namely, saline containing 12 mg of galactosamine (Wako), 0.1 $\mu$g of LPS (E. coli•0III:B12, LIST Biological Laboratories Inc.), and 200 $\mu$g or 800 $\mu$g of peptide (2) was intraperitoneally injected to 8 week-old C57BL/6 mice (females, weight 16 to 20g) (8 animals per group).

After 24 hours, the number of survived animals was counted, and the result is shown in Table 6.

Table 6

| Effect on mouse endotoxin shock model (1) | |
| --- | --- |
| Compound tested | Survived animals/observed animals |
| Peptide (2) (200 $\mu$g/mouse)<br>Peptide (2) (800 $\mu$g/mouse) | 8/8<br>8/8 |
| Physiological saline | 1/8 |

As can be seen from Table 6, the present peptide (2) has a good survival effect in the mouse endotoxin shock model, and therefore the peptide is effective on DIC, and especially on the septicshock.

2) According to the same procedure as in 1), 12 mg of galactosamine (Wako) and 0.15 $\mu$g of LPS were intraperitoneally administrated, and immediately after that a physiological saline containing 200 $\mu$g of the peptide (2) was intravenously injected (8 animals per group).

Table 7

| Effect on mouse endotoxin shock model (2) | | |
| --- | --- | --- |
| Compound tested | Administration route | Survived animals/observed animals |
| Peptide (2) (200 $\mu$g/mouse)<br>Peptide (2) (200 $\mu$g/mouse) | Simultaneous intraperitoneal<br>Intraveneous | 8/8<br>2/8 |
| Physiological saline | Intraveneous | 0/8 |

INDUSTRIAL APPLICABILITY

Peptides of the present invention have an activity to inhibit an action of phospholipase $A_2$, and promise to be useful as antiinflammatory drugs and antiallergy drugs.

SEQUENCE LISTING

SEQ ID NO: 1
Sequence length: 5
Sequence Type: Amino acid
Topology: Linear
Molecule Type: Peptide
Sequence
Lys Arg Gly Cys Gly
1                   5


SEQ ID NO: 2
Sequence length: 5
Sequence Type: Amino acid
Topology: Linear
Molecule Type: Peptide
Sequence
Lys Arg Arg Ala Gly
1                   5


SEQ ID NO: 3
Sequence length: 6
Sequence Type: Amino acid
Topology: Linear
Molecule Type: Peptide
Sequence
Lys Ala Thr Asp Ala Asn
1                   5


SEQ ID NO: 4
Sequence length: 6
Sequence Type: Amino acid
Topology: Linear
Molecule Type: Peptide
Sequence
Lys Ala Thr Asp Cys Asn
1                   5

SEQ ID NO: 5
Sequence length: 8
Sequence Type: Amino acid
Topology: Linear
Molecule Type: Peptide
Sequence
Thr Lys Phe Leu Ser Tyr Lys Phe
    1               5


SEQ ID NO: 6
Sequence length: 8
Sequence Type: Amino acid
Topology: Linear
Molecule Type: Peptide
Sequence
Thr Lys Thr Val Ser Tyr Thr Phe
    1               5


SEQ ID NO: 7
Sequence length: 8
Sequence Type: Amino acid
Topology: Linear
Molecule Type: Peptide
Sequence
Pro Lys Thr Val Ser Tyr Thr Tyr
    1               5


SEQ ID NO: 8
Sequence length: 15
Sequence Type: Amino acid
Topology: Linear
Molecule Type: Peptide
Sequence
Lys Arg Gly Cys Gly Thr Lys Phe Leu Ser Tyr Lys Phe Ser Cys
    1               5                       10                      15


SEQ ID NO: 9

Sequence length: 14
Sequence Type: Amino acid
Topology: Linear
Molecule Type: Peptide
Sequence
Lys Arg Gly Cys Gly Thr Lys Phe Leu Ser Tyr Lys Phe Ser
1               5               10

SEQ ID NO: 10
Sequence length: 15
Sequence Type: Amino acid
Topology: Linear
Molecule Type: Peptide
Sequence
Lys Arg Gly Cys Gly Thr Lys Thr Val Ser Tyr Thr Phe Ser Cys
1               5               10              15

SEQ ID NO: 11
Sequence length: 14
Sequence Type: Amino acid
Topology: Linear
Molecule Type: Peptide
Sequence
Lys Arg Gly Cys Gly Thr Lys Thr Val Ser Tyr Thr Phe Ser
1               5               10

SEQ ID NO: 12
Sequence length: 15
Sequence Type: Amino acid
Topology: Linear
Molecule Type: Peptide
Sequence
Lys Arg Gly Cys Gly Pro Lys Thr Val Ser Tyr Thr Phe Tyr Cys
1               5               10              15

SEQ ID NO: 13
Sequence length: 14

Sequence Type: Amino acid

Topology: Linear

Molecule Type: Peptide

Sequence

Lys Arg Gly Cys Gly Pro Lys Thr Val Ser Tyr Thr Tyr Ser
1               5                   10

(SEQ ID NO: 13)


SEQ ID NO: 14

Sequence length: 15

Sequence Type: Amino acid

Topology: Linear

Molecule Type: Peptide

Sequence

Lys Arg Arg Ala Gly Thr Lys Phe Leu Ser Tyr Lys Phe Ser Cys
1               5                   10                  15


SEQ ID NO: 15

Sequence length: 15

Sequence Type: Amino acid

Topology: Linear

Molecule Type: Peptide

Sequence

Lys Arg Arg Ala Gly Thr Lys Thr Val Ser Tyr Thr Phe Ser Cys
1               5                   10                  15


SEQ ID NO: 16

Sequence length: 15

Sequence Type: Amino acid

Topology: Linear

Molecule Type: Peptide

Sequence

Lys Arg Arg Ala Gly Pro Lys Thr Val Ser Tyr Thr Phe Tyr Cys
1               5                   10                  15


SEQ ID NO: 17

Sequence length: 16

Sequence Type: Amino acid

Topology: Linear

Molecule Type: Peptide

Sequence

Lys Ala Thr Asp Ala Asn Thr Lys Phe Val Ser Tyr Thr Phe Ser Cys
    1               5                   10                  15


SEQ ID NO: 18

Sequence length: 16

Sequence Type: Amino acid

Topology: Linear

Molecule Type: Peptide

Sequence

Lys Ala Thr Asp Ala Asn Thr Lys Thr Val Ser Tyr Thr Phe Ser Cys
    1               5                   10                  15


SEQ ID NO: 19

Sequence length: 16

Sequence Type: Amino acid

Topology: Linear

Molecule Type: Peptide

Sequence

Lys Ala Thr Asp Ala Asn Pro Lys Thr Val Ser Tyr Thr Phe Tyr Cys
    1               5                   10                  15


SEQ ID NO: 20

Sequence length: 16

Sequence Type: Amino acid

Topology: Linear

Molecule Type: Peptide

Sequence

Lys Ala Thr Asp Cys Asn Thr Lys Phe Leu Ser Tyr Lys Phe Ser Cys
    1               5                   10                  15


SEQ ID NO: 21

Sequence length: 15

Sequence Type: Amino acid

Topology: Linear

Molecule Type: Peptide

Sequence

Lys Ala Thr Asp Cys Asn Thr Lys Phe Leu Ser Tyr Lys Phe Ser
1                   5                   10                  15


SEQ ID NO: 22

Sequence length: 16

Sequence Type: Amino acid

Topology: Linear

Molecule Type: Peptide

Sequence

Lys Ala Thr Asp Cys Asn Thr Lys Thr Val Ser Tyr Thr Phe Ser Cys
1                   5                   10                  15


SEQ ID NO: 23

Sequence length: 15

Sequence Type: Amino acid

Topology: Linear

Molecule Type: Peptide

Sequence

Lys Ala Thr Asp Cys Asn Thr Lys Thr Val Ser Tyr Thr Phe Ser
1                   5                   10                  15


SEQ ID NO: 24

Sequence length: 16

Sequence Type: Amino acid

Topology: Linear

Molecule Type: Peptide

Sequence

Lys Ala Thr Asp Cys Asn Pro Lys Thr Val Ser Tyr Thr Tyr Ser Cys
1                   5                   10                  15


SEQ ID NO: 25

Sequence length: 15

Sequence Type: Amino acid

Topology: Linear

```
Molecule Type: Peptide
Sequence
Lys Ala Thr Asp Cys Asn Pro Lys Thr Val Ser Tyr Thr Tyr Ser
 1               5                   10                  15
```

**Claims**

1. A physiologically active peptide represented by the following formula (I):

   A-W$^1$-X-V$^1$-Z-W$^2$-Y$^1$-V$^2$-Y$^2$-B     (I)

   wherein A represents a peptide consisting of 1 to 6 amino acid residues; B represents a peptide consisting of 1 to 3 amino acid residues; W$^1$ and W$^2$ represent, independently, Ser or Thr; X represents Lys or Arg; V$^1$ and V$^2$ represent, independently, Ser, Arg, Thr, Tyr, Phe or Lys; Y$^1$ and Y$^2$ represent, independently, Phe, Tyr or Trp; and Z represents Leu, Val, Ile, Pro, Phe or Ala; at least one of A and B contains at least one Cys residue; and wherein
   
   (1) in a peptide containing at least one Cys residue in A and/or B, said at least one Cys residue is modified at its -SH group;
   
   (2) in a peptide containing at least one Cys residue in both of A and B, a ring structure has been formed by a disulfide bond between said two Cys residues;
   
   (3) in more than one peptide each containing at least one Cys residue in at least one of A and/or B, a linear polymer has been formed by one or more disulfide bond formed between a Cys residue contained in A or B of one peptide and a Cys residue contained in A or B of another peptide;
   
   (4) in more than one peptide each containing at least one Cys residue in both of A and B, polymeric ring structure has been formed by more than one disulfide bond formed between a Cys residue contained in A or B of a peptide and Cys residue contained in A or B of another peptide; or
   
   (5) in the structures (1) to (4), at least one Cys residue which does not form a disulfide bond has been modified at its -SH group.

2. A physiologically active peptide according to claim 1, wherein the polymer is a dimer.

3. A physiologically active peptide according to claim 1, wherein at least A contains a Cys residue, and the -SH group of the Cys residue is modified.

4. A physiologically active peptide according to claim 1, wherein at least B contains a Cys residue, and the -SH group of the Cys residue is modified.

5. A physiologically active peptide according to claim 1, wherein each of A and B contains a Cys residue, and the -SH group of the Cys residue is modified.

6. A physiologically active peptide according to claim 1, which is a dimer consisting of two peptides each containing a Cys residue in at least A wherein said two Cys residues form one disulfide bond.

7. A physiologically active peptide according to claim 1, which is a dimer consisting of two peptides each containing a Cys residue in at least B wherein said two Cys residues form one disulfide bond.

8. A physiologically active peptide according to claim 1, which is a dimer consisting of a peptide containing a Cys residue in at least A and a peptide containing a Cys residue in at least B wherein said two Cys residues form one disulfide bond.

9. A physiologically active peptide according to claim 1, consisting of two peptides each containing Cys residues in A and B, and having a dimer ring structure formed by a disulfide bond formed from a Cys residue contained in A of a peptide and a Cys residue in A of another peptide, and a disulfide bond formed from a Cys residue contained in B of a peptide and Cys residue contained in B of another

29

peptide.

10. A physiologically active peptide according to claim 1, consisting of two peptides each containing Cys residues in A and B, and having a dimer ring structure formed by two disulfide bonds each formed from a Cys residue contained in A of a peptide and a Cys residue contained in B of another peptide.

11. A physiologically active peptide according to claim 1 wherein in the formula (I), A has a Cys residue and consists of 5 or 6 amino acid residues selected from the group consisting of Lys, Arg, Gly, Ala, Asn, Gln, Ser, Thr, Asp, Glu and Cys.

12. A physiologically active peptide according to claim 1, wherein in the formula (I), B has a Cys residue and consisting of 2 amino acid residues selected from the group consisting of Ser, Thr and Cys.

13. A physiologically active peptide according to claim 1, wherein in the formula (I), $V^1$ and $V^2$ are independently from each other Phe or Lys.

14. A physiologically active peptide according to claim 1, wherein in the formula (I), $Y^1$ and $Y^2$ are, independently from each other Phe or Tyr.

15. A physiologically active peptide according to claim 1, wherein in the formula (I), Z is Leu.

16. A physiologically active peptide according to claim 1, wherein in the formula (I), there are replacement, deletion and/or insertion of one or more amino acid residues.

17. A pharmaceutical composition comprising a physiologically active peptide according to any one of claims 1 to 16 and a pharmaceutically active carrier.

18. A pharmaceutical composition according to claim 17, which is a phospholipase $A_2$ inhibitor.

19. A pharmaceutical composition according to claims 17 or 18, which is a prophylactic and/or therapeutic agent for inflammatory diseases, allergy diseases, pancreatitis, increase of severity of pancreatitis and/or multi-organ diseases.

# Fig. 1

# Fig. 2

# Fig.3

# Fig.4

# Fig.5

# Fig.6

# Fig.7

# Fig.8

34

# Fig.9

| A. | CLASSIFICATION OF SUBJECT MATTER |
|---|---|

Int. Cl$^5$  C07K7/06, 7/08, A61K37/64

According to International Patent Classification (IPC) or to both national classification and IPC

| B. | FIELDS SEARCHED |
|---|---|

Minimum documentation searched (classification system followed by classification symbols)

Int. Cl$^5$  C07K7/00, A61K37/00

Documentation searched other than minimum documentation to the extent that such documents are included in the fields searched

Electronic data base consulted during the international search (name of data base and, where practicable, search terms used)

CAS ONLINE

| C. | DOCUMENTS CONSIDERED TO BE RELEVANT | |
|---|---|---|

| Category* | Citation of document, with indication, where appropriate, of the relevant passages | Relevant to claim No. |
|---|---|---|
| A | J. Biol. Chem., Vol. 252, No. 14 (1977) Robert L. Heinrikson, et al "Amino acid sequence of phospholipase A2-alpha from the venom of Crotalus adamanteus. A new classification of phospholipases A2 based upon structural determinants." PP. 4913-4921 | 1-19 |
| A | J. Biol. Chem., Vol 264, No. 10 (1989) Ruth M. Kramer, et al "Structure and properties of a human non-pancreatic phospholipase A2" PP. 5768-5775 | 1-19 |

☑ Further documents are listed in the continuation of Box C.  ☐ See patent family annex.

| * | Special categories of cited documents: |
|---|---|
| "A" | document defining the general state of the art which is not considered to be of particular relevance |
| "E" | earlier document but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| May 17, 1994 (17. 05. 94) | June 14, 1994 (14. 06. 94) |

| Name and mailing address of the ISA/ | Authorized officer |
|---|---|
| Japanese Patent Office | |
| Facsimile No. | Telephone No. |

Form PCT/ISA/210 (second sheet) (July 1992)